# EUROPEAN PATENT APPLICATION

(11) **EP 3 315 088 A1**
(43) Date of publication of application: **02.05.2018**
(21) Application number: 15897071.5
(22) Date of filing: 29.06.2015
(51) Int. Cl.: A61B 34/00, B25J 9/06

(54) **SURGICAL ROBOT**

(71) Applicant: Kawasaki Jukogyo Kabushiki Kaisha, Kobe-shi, Hyogo 650-8670 (JP)
(72) Inventor: HASHIMOTO, Yasuhiko, Akashi-shi Hyogo 673-8666 (JP)
(74) Representative: Witte, Weller & Partner Patentanwälte mbB
(86) International application number: PCT/JP2015/003252
(87) International publication number: WO 2017/002143

(57) **Abstract**

A surgical robot includes: a base disposed under a surgical table; and a plurality of multi-jointed arms extending upward from the base and passing beside the surgical table, the multi-jointed arms being configured such that a medical tool is mountable to a distal end of each of the multi-jointed arms, the multi-jointed arms each having a plurality of degrees of freedom.

## Description

### Technical Field

The present invention relates to a surgical robot.

### Background Art

Conventionally, there are known surgical robots that are remote-controlled by surgeons. For example, Patent Literature 1 discloses a surgical robot including: a tower-shaped support frame installed upright next to a surgical table; and a plurality of arms extending laterally from the upper part of the support frame. Various types of medical tools are mountable to the distal ends of the arms.

### Citation List

### Patent Literature

PTL 1: Japanese Laid-Open Patent Application Publication No. 2015-502768

### Summary of Invention

### Technical Problem

However, in the case of the surgical robot disclosed in Patent Literature 1, since the tower-shaped large support frame is installed upright next to the surgical table, not only does the surgical robot cause surgical staff such as assistant surgeons and nurses to feel cramped, the surgical robot also significantly limits the area of movement of the surgical staff around the surgical table.

In view of the above, an object of the present invention is to provide a surgical robot that hardly becomes an obstacle for surgical staff.

### Solution to Problem

In order to solve the above-described problems, a surgical robot of the present invention includes: a base disposed under a surgical table; and a plurality of multi-jointed arms extending upward from the base and passing beside the surgical table, the multi-jointed arms being configured such that a medical tool is mountable to a distal end of each of the multi-jointed arms, the multi-jointed arms each having a plurality of degrees of freedom.

According to the above configuration, only the multi-jointed arms are present at the sides of and above the surgical table, and the base of the surgical robot is concealed under the surgical table. Therefore, the surgical robot would neither cause the surgical staff to feel cramped, nor significantly limit the area of movement of the surgical staff around the surgical table. Thus, the present invention makes it possible to realize the surgical robot that hardly becomes an obstacle for the surgical staff.

The plurality of multi-jointed arms may be configured to be retractable under the surgical table. According to this configuration, when the use of the surgical robot is unnecessary, the multi-jointed arms can be retracted under the surgical table, and the entire surgical robot can be stored under the surgical table.

The above surgical robot may further include a robot control board built in the base, the robot control board being configured such that: when the robot control board receives a use start signal, the robot control board controls the plurality of multi-jointed arms, such that the plurality of multi-jointed arms move to an origin position for a surgery; and when the robot control board receives a use end signal, the robot control board controls the plurality of multi-jointed arms, such that the plurality of multi-jointed arms are retracted under the surgical table. According to this configuration, the movement of the multi-jointed arms to the origin position for a surgery and the retraction of the multi-jointed arms under the surgical table can be automated.

The base may be configured to be movable in a longitudinal direction of the surgical table. According to this configuration, the base of the surgical robot can be disposed at a suitable position in accordance with, for example, the position of a patient on the surgical table and a surgical site of the patient.

### Advantageous Effects of Invention

The present invention provides a surgical robot that hardly becomes an obstacle for surgical staff.

### Brief Description of Drawings

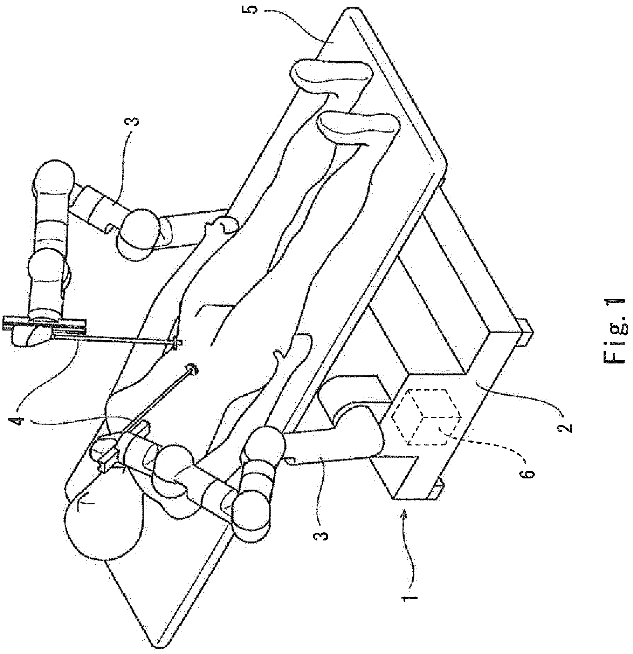

Fig. 1 is a perspective view of a surgical robot and a surgical table according to one embodiment of the present invention.

### Description of Embodiments

Fig. 1 shows a surgical robot 1 according to one embodiment of the present invention. The surgical robot 1 is connected to a master controller (not shown) via a cable, and is remote-controlled by a surgeon. That is, the master controller (not shown) and the surgical robot 1 form a master-slave system.

Specifically, the surgical robot 1 includes: a plurality of multi-jointed arms 3 configured such that a medical tool 4 is mountable to the distal end of each of the multi-jointed arms 3; and a base 2 configured to support these multi-jointed arms 3. In the present embodiment, the number of multi-jointed arms 3 is two. However, as an alternative, the number of multi-jointed arms 3 may be three or more.

Examples of the medical tool 4 mountable to the distal end of each of the multi-jointed arms 3 include forceps, an aspirator, a laparoscopic camera, and a surgical knife. The two multi-jointed arms 3 are controlled by a robot control board 6 built in the base 2.

The base 2 is box-shaped and has a relatively low height. The height of the base 2 is set to be lower than the height of a surgical table 5, on which a patient lies down. The base 2 is disposed under the surgical table 5. Desirably, in the width direction of the surgical table 5, the width of the base 2 is smaller than the width of the surgical table 5.

The surgical table 5 may be supported by a support pillar installed upright on a floor on which the surgical robot 1 is placed. In this case, the surgical table 5 may be coupled to the base 2. Alternatively, the surgical table 5 may be supported by the base 2.

The base 2 may be fixed to the floor. However, desirably, the base 2 is configured to be movable in the longitudinal direction of the surgical table 5, because with such a configuration, the base 2 of the surgical robot 1 can be disposed at a suitable position in accordance with, for example, the position of the patient on the surgical table 5 and a surgical site of the patient. For example, a running device including servomotors configured to drive a plurality of wheels is incorporated in the base 2. In this case, the surgical robot 1 is moved by controlling the servomotors by the robot control board 6. However, as an alternative, the base 2 may be transferred in the longitudinal direction of the surgical table 5 by a transfer mechanism (e.g., a ball screw mechanism) provided on the floor.

The two multi-jointed arms 3 extend upward from the base 2, passing beside the surgical table 5. In the present embodiment, the multi-jointed arms 3 are coupled to both ends of the base 2, respectively, in the width direction of the surgical table 5, and extend upward passing both sides of the surgical table 5. However, as an alternative, the two multi-jointed arms 3 may be coupled to one end of the base 2 in the width direction of the surgical table 5, and may extend upward passing only one side of the surgical table 5.

Each multi-jointed arm 3 has a plurality of degrees of freedom. Desirably, each multi-jointed arm 3 has at least six degrees of freedom (e.g., seven degrees of freedom).

Desirably, the two multi-jointed arms 3 are configured to be retractable under the surgical table 5, because with such a configuration, when the use of the surgical robot 1 is unnecessary, the multi-jointed arms 3 can be retracted under the surgical table 5, and the entire surgical robot 1 can be stored under the surgical table 5.

For example, when the robot control board 6 receives a use start signal, the robot control board 6 may control the two multi-jointed arms 3, such that the two multi-jointed arms 3 move to the origin position for a surgery. Also, when the robot control board 6 receives a use end signal, the robot control board 6 may control the two multi-jointed arms 3, such that the two multi-jointed arms 3 are retracted under the surgical table 5. According to this configuration, the movement of the multi-jointed arms 3 to the origin position for a surgery and the retraction of the multi-jointed arms 3 under the surgical table 5 can be automated. It should be noted that each of the use start signal and the use end signal may be, for example, a sound signal from a microphone or an ON/OFF signal from a switch. Such a microphone and a switch may be provided on the master controller, which is not shown, or may be provided on the base 2.

As described above, the surgical robot 1 of the present embodiment is configured such that only the multi-jointed arms 3 are present at the sides of and above the surgical table 5, and the base 2 of the surgical robot 1 is concealed under the surgical table 5. Therefore, the surgical robot 1 would neither cause the surgical staff such as assistant surgeons and nurses to feel cramped, nor significantly limit the area of movement of the surgical staff around the surgical table 5. Thus, the present invention makes it possible to realize the surgical robot 1, which hardly becomes an obstacle for the surgical staff.

It should be noted that the present invention is not limited to the above-described embodiment. Various modifications can be made without departing from the spirit of the present invention.

### Reference Signs List

1 surgical robot
2 base
3 multi-jointed arm
4 medical tool
5 surgical table
6 robot control board

## Claims

1. A surgical robot comprising:
a base disposed under a surgical table; and
a plurality of multi-jointed arms extending upward from the base and passing beside the surgical table, the multi-jointed arms being configured such that a medical tool is mountable to a distal end of each of the multi-jointed arms, the multi-jointed arms each having a plurality of degrees of freedom.

2. The surgical robot according to claim 1, wherein
the plurality of multi-jointed arms are configured to be retractable under the surgical table.

3. The surgical robot according to claim 2, further comprising a robot control board built in the base, the robot control board being configured such that:
when the robot control board receives a use start signal, the robot control board controls the plurality of multi-jointed arms, such that the plurality of multi-jointed arms move to an origin position for a surgery; and
when the robot control board receives a use end signal, the robot control board controls the plurality of multi-jointed arms, such that the plurality of multi-jointed arms are retracted under the surgical table.

4. The surgical robot according to any one of claims 1 to 3, wherein
the base is configured to be movable in a longitudinal direction of the surgical table.
